(19) Europäisches Patentamt
European Patent Office
Office européen des brevets

(11) Veröffentlichungsnummer: **0 019 744**
**B1**

(12) # EUROPÄISCHE PATENTSCHRIFT

(45) Veröffentlichungstag der Patentschrift: **29.12.82**

(51) Int. Cl.³: **C 07 C 79/26,** C 07 C 76/02

(21) Anmeldenummer: **80102416.7**

(22) Anmeldetag: **05.05.80**

(54) **Verfahren zur Herstellung von Chlorameisensäure-(3-nitro-4-methyl)-phenylester.**

(30) Priorität: **19.05.79 DE 2920386**

(43) Veröffentlichungstag der Anmeldung:
**10.12.80 Patentblatt 80/25**

(45) Bekanntmachung des Hinweises auf die Patenterteilung:
**29.12.82 Patentblatt 82/52**

(84) Benannte Vertragsstaaten:
**BE CH DE FR GB IT LI**

(56) Entgegenhaltungen:
**DE-A-2 213 568**
**DE-C-206 638**
**GB-A-941 709**
**Patents Abstracts of Japan Band 2, Nr. 8, 20. Januar 1978 Seite 3818C77**
**HOUBEN—WEYL »Methoden der Organischen Chemie«, Band 10/1, Teil 1, 1971, GEORG THIEME VERLAG, Stuttgart, Seite 620**

(73) Patentinhaber: **BASF Aktiengesellschaft, Carl-Bosch-Strasse 38, D-6700 Ludwigshafen (DE)**

(72) Erfinder: **Scheuermann, Horst, Dr., Bexbacher Strasse 41, D-6700 Ludwigshafen (DE)**
Erfinder: **Schneider, Dieter, Dr., Neumannstrasse 18, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 744 B1

BUNDESDRUCKEREI BERLIN

Verfahren zur Herstellung von Chlorameisensäure-(3-nitro-4-methyl)-phenylester

Die Erfindung betrifft ein Verfahren zur Herstellung von Chlorameisensäure-(3-nitro-4-methyl)-phenylester durch Umsetzung von Chlorameisensäure-(4-methyl)-phenylester mit Salpetersäure in Gegenwart von Schwefelsäure.

Es ist aus Houben-Weyl, Methoden der Organischen Chemie, Band 10/1, Seite 621, bekannt, daß man Benzoesäuremethylester mit Nitriersäure und Schwefelsäure zu 3-Nitrobenzoesäuremethylester umsetzen kann. Es wird angegeben, daß hingegen Benzoylchlorid als Ausgangsstoff sich ohne Hydrolyse nur mit Distickstoffpentoxid in Tetrachlormethan nitrieren läßt. Jedoch ist die übliche Herstellung die Überführung der leichter zugänglichen nitrierten Benzoesäuren in das Säurechlorid.

Die DE-C-206 638 beschreibt die Herstellung von 1-Methyl-2-nitro-4-oxybenzol durch Behandlung von p-Kresolcarbonat mit nitrierenden Mitteln und Verseifung des so erhaltenen o-Nitro-p-kresolcarbonats. Nachteilig sind die Arbeitsweise in zwei Stufen und somit umständliche Reaktionen und Aufarbeitung.

Die DE-A-2 213 568 zeigt die Herstellung von 3-Nitro-p-kresol-(1) durch Umsetzung von p-Kresolcarbonat mit Salpetersäure, Fällung des gebildeten 3-Nitro-p-kresolcarbonats bei 85 bis 100°C durch Verdünnen mit Wasser und Verseifung des Carbonats in wäßrigem Ammoniak bei 65 bis 80°C. Nachteilig sind auch hier die verschiedenen Umsetzungs- und Reinigungsstufen.

Auch das in JP-A-52 111 539 (Patent Abstracts of Japan Vol. 2, Nr. 8 vom 20. 01. 78) beschriebene Verfahren, benötigt organische Lösungsmittel und Hilfsbasen bei der Umsetzung von p-Nitrophenol mit Phosgen in Gegenwart von N,N-Diethylanilin.

Es wurde nun gefunden, daß man Chlorameisensäure-(3-nitro-4-methyl)-phenylester vorteilhaft erhält, wenn man Chlorameisensäure-(4-methyl)-phenylester mit Salpetersäure in Gegenwart von Schwefelsäure umsetzt.

Die Umsetzung kann durch die folgenden Formeln wiedergegeben werden:

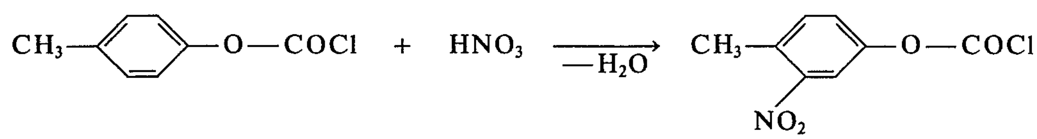


Das Verfahren nach der Erfindung liefert auf einfachem und wirtschaftlichem Wege den Chlorameisensäure-(3-nitro-4-methyl)-phenylester in guter Ausbeute und Reinheit. Alle diese vorteilhaften Ergebnisse sind im Hinblick auf den Stand der Technik überraschend.

Man nitriert mit Salpetersäure, vorteilhaft konzentrierter oder rauchender Salpetersäure, in Gegenwart von Schwefelsäure, vorteilhaft hochkonzentrierter oder rauchender Schwefelsäure. Gegebenenfalls kann man ganz oder teilweise Salpetersäure und gewünschtenfalls Schwefelsäure durch Nitriersäure, einem Gemisch der beiden Säuren, ersetzen. Im allgemeinen verwendet man eine 85- bis 100gewichtsprozentige Salpetersäure und/oder eine 98- bis 100gewichtsprozentige Schwefelsäure. Zweckmäßig wählt man in dem Gemisch von Salpetersäure und Schwefelsäure oder der Nitriersäure ein Verhältnis von 0,2 bis 2 Mol Salpetersäure je Mol Schwefelsäure. In der Regel verwendet man von 35 bis 50, vorzugsweise von 40 bis 45 Gewichtsprozent Salpetersäure, bezogen auf Chlorameisensäure-(4-methyl)-phenylester. Bevorzugt ist eine Menge von Schwefelsäure von 200 bis 1500, bevorzugt 400 bis 700 Gewichtsprozent, bezogen auf Chlorameisensäure-(4-methyl)-phenylester. Bevorzugt ist eine Menge von Nitriersäure von 70 bis 95, bevorzugt 75 bis 85 Gewichtsprozent, bezogen auf Chlorameisensäure-(4-methyl)-phenylester. Anstelle von Salpetersäure können auch Stoffe, die diese Säure im Reaktionsgemisch bilden, z. B. anorganische Nitrate wie Natrium- oder Kaliumnitrat, in entsprechenden Mengen zur Anwendung gelangen.

Gegebenenfalls verwendet man Harnstoff als Nitrierkatalysator, zweckmäßig in einer Menge von 0,05 bis 100, vorzugsweise von 5 bis 10 Gewichtsprozent, bezogen auf Chlorameisensäure-(4-methyl)-phenylester. Die Umsetzung wird im allgemeinen bei einer Temperatur von −10°C bis +40°C, vorzugsweise von −10°C bis +25°C, insbesondere von 0°C bis 5°C, drucklos oder unter Druck, diskontinuierlich oder kontinuierlich durchgeführt. Lösungsmedium ist die Säure bzw. das Säuregemisch im allgemeinen selbst, gegebenenfalls im Gemisch mit Wasser als entsprechend konzentriertes Säuregemisch.

Die Reaktion kann wie folgt durchgeführt werden: Ein Gemisch von Ausgangsstoff und Schwefelsäure wird auf die Reaktionstemperatur gebracht und dann unter Rühren langsam, z. B. während 15 bis 30 Minuten, mit Salpetersäure, gegebenenfalls im Gemisch mit Schwefelsäure, versetzt. Das Gemisch wird noch 0,5 bis 10, vorzugsweise 0,5 bis 1,5 Stunden bei der Reaktionstemperatur gehalten. In entsprechender Weise kann die Reaktion auch kontinuierlich durchgeführt werden, indem man z. B. einem Reaktionsrohr getrennt das vorgenannte schwefelsaure Gemisch und die Salpetersäure zuführt und beide Komponenten dort in dünnem Strahl miteinander gut vermischt. Zweckmäßig beläßt man das Reaktionsgemisch 5 bis 30 Minuten im Rohr bei der

Reaktionstemperatur und führt dann die Aufarbeitung durch. Gegebenenfalls kann man inerte Gase, z. B. Stickstoff, durch den Reaktionsraum leiten, um gebildete nitrose Gase zu entfernen.

Der Endstoff kann dann aus dem Reaktionsgemisch in üblicher Weise, z. B. durch Fällung auf Eis, zweckmäßig in einer Menge von 100 bis 1000, bevorzugt 200 bis 300 Gewichtsprozent Eis, bezogen auf Schwefelsäure Monohydrat, und Filtration, isoliert werden. Anstelle der Isolierung des reinen Endstoffs kann man auch das Rohprodukt nach erneutem Digerieren in Wasser bei 85 bis 98°C hydrolysieren. Nach Abkühlen und Absaugen erhält man so das rohe 3-Nitro-4-methylphenol in über 87prozentiger Ausbeute, bezogen auf den Chlorameisensäure-(4-methyl)-phenylester. Es enthält etwa 2,5 Gewichtsprozent 2-Nitro-4-methylphenol. Um reines 3-Nitro-4-methylphenol zu erhalten, kann man bei der Hydrolyse das Isomere mit Wasserdampf abdestillieren. Man hydrolysiert den feuchten Endstoff zweckmäßig in siedendem Wasser in der 1,5- bis 10fachen, bevorzugt 3- bis 4fachen Menge, bezogen auf den Ausgangsstoff, unter gleichzeitigem Abdestillieren von Wasserdampf und unerwünschtem 2-Nitro-4-methylphenol.

Der neue Chlorameisensäure-(3-nitro-4-methyl)-phenylester ist ein wertvoller Ausgangsstoff für die Herstellung von Farbstoffen und Pflanzenschutzmitteln, insbesondere ein Zwischenprodukt für 3-Nitro-4-methylphenol, das zum 3-Amino-4-methylphenol reduziert wird. 3-Amino-4-methylphenol und seine Derivate sind zur Herstellung von Farbstoffen von Interesse (Ullmanns Encyklopädie der technischen Chemie, Band 13, Seite 8).

Die in den folgenden Beispielen angegebenen Teile bedeuten Gewichtsteile.

## Beispiel 1

a) 85 Teile Chlorameisensäure-(4-methyl)-phenylester werden zwischen 0 und 10°C zu 340 Teilen Monohydrat langsam zugegeben. Bei 3°C dosiert man 68 Teile Mischsäure, die 52 Teile Salpetersäure und 48 Teile Schwefelsäure je 100 Teile enthält, zu. Man rührt das Gemisch eine Stunde nach und gießt es dann auf 1500 Teile Eis. Nach 1/2stündigem Rühren saugt man das Gemisch ab, wäscht das Filtergut mit Eiswasser neutral und erhält nach Trocknen 94 Teile Chlorameisensäure-(3-nitro-4-methyl)-phenylester, entsprechend 87% der Theorie. Der Endstoff enthält 2,5 Gewichtsprozent Chlorameisensäure-(2-nitro)-4-methyl)-phenylester. Durch Umkristallisation aus Cyclohexan erhält man in 82,5% der Theorie den reinen Endstoff vom Fp 39 bis 42°C.

b) Man setzt analog Beispiel 1a) um, aber digeriert nach dem Ausfällen auf Eis und Absaugen das Rohprodukt in 300 Teilen Wasser. Man erhitzt das Gemisch zum Sieden und destilliert unter Einblasen von Wasserdampf das 2-Nitro-4-methyl-phenol ab. Danach kühlt man auf 25°C, saugt das ausgefallene 3-Nitro-4-methylphenol ab und trocknet es bei 40°C (20 mbar). Man erhält 65 Teile 3-Nitro-4-methylphenol, entsprechend 85% der Theorie, bezogen auf Chlorameisensäure-(4-methyl)-phenylester, vom Fp 75°C.

## Beispiel 2

Man setzt analog Beispiel 1b) um, führt aber die Nitrierung mit 35 Teilen 98gewichtsprozentiger Salpetersäure durch. Die Ausbeute beträgt 60 Teile 3-Nitro-4-methylphenol, entsprechend 78% der Theorie, vom Fp 72°C.

Arbeitet man das Reaktionsgemisch analog Beispiel 1a) auf, so erhält man 79,8 Teile (74,1% der Theorie) Chlorameisensäure-(2-nitro-4-methyl)-phenylester vom Fp 40 bis 42°C.

## Beispiel 3

85 Teile Chlorameisensäure-(4-methyl)-phenylester werden zwischen 0 und 10°C zu 400 Teilen Monohydrat langsam zugegeben. Bei 3°C trägt man 56 Teile Kaliumnitrat innerhalb von 2 Stunden ein. Man rührt das Gemisch 2 Stunden nach und fällt es dann auf 2000 Teile Eis. Nach Aufarbeitung gemäß Beispiel 1b) erhält man 64 Teile 3-Nitro-4-methylphenol, entsprechend 84% der Theorie, bezogen auf Chlorameisensäure-(4-methyl)-phenylester, vom Fp 74°C.

Arbeitet man das Reaktionsgemisch analog Beispiel 1a) auf, so erhält man 81,4 Teile (75,6% der Theorie) Chlorameisensäure-(2-nitro-4-methyl)-phenylester vom Fp 38 bis 41°C.

## Beispiel 4

Man setzt analog Beispiel 1b) um, aber verwendet hier statt Monohydrat 575 Teile Schwefelsäure (98 Gew.-%). Man erhält 48,8 Teile 3-Nitro-4-methylphenol, entsprechend 64% der Theorie, bezogen auf Chlorameisensäure-(4-methyl)-phenylester, vom Fp 75°C.

Arbeitet man das Reaktionsgemisch analog Beispiel 1a) auf, so erhält man 62 Teile (57,6% der Theorie) Chlorameisensäure-(2-nitro-4-methyl)-phenylester vom Fp 40 bis 42°C.

Beispiel 5

Man führt die Umsetzung analog Beispiel 1a) durch, arbeitet aber nach der Nitrierung wie folgt auf: Das Reaktionsgemisch wird auf 1500 Teile Wasser gegeben und mit Wasserdampf destilliert, wobei gleichzeitig der rohe Chlorameisensäure-(3-nitro-4-methyl)-phenylester hydrolysiert und 2-Nitro-4-methyl-phenol abdestilliert wird. Man kühlt auf 25° C, saugt das ausgefallene 3-Nitro-4-methylphenol ab und trocknet es bei 40° C (20 mbar). Man erhält 68 Teile 3-Nitro-4-methylphenol, entsprechend 89% der Theorie, bezogen auf Chlorameisensäure-(4-methyl)-phenylester, vom Fp 73 bis 76° C.

**Patentanspruch**

Verfahren zur Herstellung von Chlorameisensäure-(3-nitro-4-methyl)-phenylester, dadurch gekennzeichnet, daß man Chlorameisensäure-(4-methyl)-phenylester mit Salpetersäure in Gegenwart von Schwefelsäure umsetzt.

**Claim**

A process for the preparation of (3-nitro-4-methyl)-phenyl chloroformate, wherein (4-methyl)-phenyl chloroformate is reacted with nitric acid in the presence of sulfuric acid.

**Revendication**

Procédé de préparation du chloroformiate de nitro-3 méthyl-4 phényle, caractérisé en ce que l'on fait réagir le chloroformiate de méthyl-4 phényle en présence d'acide sulfurique avec de l'acide nitrique.